# EUROPEAN PATENT APPLICATION

(11) **EP 4 696 134 A2**
(43) Date of publication of application: **18.02.2026**
(21) Application number: 25215886.0
(22) Date of filing: 29.11.2023
(51) Int. Cl.: A01M 1/20, A01M 7/00, A61L 9/03

(54) **INSECT REPELLER HAVING CIRCUIT BOARD SUPPORT STRUCTURE**

(30) Priority: 30.11.2022 US 202263428937 P
(62) Divisional of application: 23898784.6
(71) Applicant: Thermacell Repellents, Inc., Bedford, MA 01730 (US)
(72) Inventor: BOURQUE, Steven, M., Billerica, 01821 (US); CASTRO, Fernando, L., Westwood, 02090 (US)
(74) Representative: Haseltine Lake Kempner LLP

(57) **Abstract**

An illuminated insect repeller includes a heater assembly, an electrical power source, a repeller power switch, a power button assembly including a status indicator lens, a repeller power button, and at least one light pipe optically connected to the status indicator lens and configured to project light from a light source to the status indicator lens. The repeller power switch is in electrical communication between the electrical power source and the heater assembly.

## Description

### CROSS-REFERENCE TO RELATED APPLICATIONS

This application claims the benefit of United States Provisional Application No. 63/428,937, filed November 30, 2022, the disclosure of which is incorporated herein by reference in its entirety.

### BACKGROUND OF THE INVENTION

This invention relates in general to insect repellers. In particular, this invention relates to an improved printed circuit board (PCB) assembly and a method for assembling the PCB assembly.

Outdoor spaces provide an attractive place for people to gather, eat, and relax. However, these spaces also attract insects and other pests which can hamper effective area utilization and, particularly in a commercial setting, create an undesirable environment which reduces profitability and adversely can impact a facility's reputation. Individual insect repellers are known and create defined areas of protection from pests. These devices work well but are independently operated and may not provide efficient utilization of volatized materials which increases costs. Other devices are known to be linked to provide power for heating elements but do not regulate operation of the devices in response to environmental conditions or system performance levels.

The ability to conveniently and effectively control an outdoor environment with respect to pest repelling or ambiance creation is hampered by the ability of insect repellers to communicate and coordinate operational status, adjust output parameters based on local conditions, and accommodate different materials and outputs to provide different environmental effects such as scent regulation, lighting control, and audio outputs. In addition, the outdoor environment provides deleterious conditions, such as rain, wind, and temperature changes that reduce the ability to efficiently use emissive materials. Thus, it would be desirable to provide an outdoor environmental control system that provides the ability to reduce pest presence, provides a pleasant sensory atmosphere, is illuminated, easier to assemble, and is resistant to negative environmental factors that affect operation of the devices.

### SUMMARY OF THE INVENTION

This invention relates in general to insect repellers. In particular, this invention relates to an improved printed circuit board (PCB) assembly and a method for assembling the PCB assembly.

In one embodiment, an illuminated insect repeller includes a heater assembly, an electrical power source, a repeller power switch, a power button assembly including a status indicator lens, a repeller power button, and at least one light pipe optically connected to the status indicator lens and configured to project light from a light source to the status indicator lens. The repeller power switch is in electrical communication between the electrical power source and the heater assembly.

In a second embodiment, a PCB assembly for an insect repeller includes a PCB mounting brace having a base and a PCB access opening formed therethrough. A PCB has a first and a second side, and has a microcontroller, one or more functional electronic components, and a power switch having an outwardly extending power switch post electrically mounted to the first side of the PCB and configured to operate the insect repeller. The PCB is mounted within a groove formed in the PCB mounting brace. A plurality of first pogo pins is mounted to a first end of the PCB. An electrical connector is mounted to a base of the PCB mounting brace, the electrical connector having a plurality of second pogo pins and electrical leads extending from the electrical connector through the PCB access opening, to the second side of the PCB. A light pipe sub-assembly includes a light pipe housing having a button opening centrally formed therein, attachment arms extending outward thereof, and a plurality of light pipes, the attachment arms extending through the PCB and into the PCB mounting brace, a power button mounted within the button opening, a status indicator lens attached to the light pipe sub-assembly, and a spring mounted between the power switch post and the power button.

Additionally, a method of assembling a PCB assembly for an insect repeller includes mounting to a first side of a PCB electrical components necessary to operate the insect repeller, including at least one of a microcontroller, one or more functional electronic components, a power button having an outwardly extending power switch post electrically mounted to the first side of the PCB and configured to operate the insect repeller. A plurality of first pogo pins is placed onto posts and the posts are heat staked to lock the plurality of pogo pins in place at a first end of the PCB. An electrical connector is mounted to a base of a PCB mounting brace, the electrical connector having a plurality of second pogo pins and pogo pin electrical leads extending from the electrical connector. The PCB is mounted into a groove formed in the PCB mounting brace and the pogo pin electrical leads from the electrical connector on the base of the brace are connected through the PCB access opening to a second side of the PCB. A light pipe sub-assembly is provided and includes a light pipe housing having a button opening centrally formed therein, attachment arms extending outward thereof, and a plurality of light pipes. A push button is snapped into the button opening of the light pipe sub-assembly. A spring is attached to the power switch post and the attachment arms of the light pipe sub-assembly are snapped through the PCB and onto to the PCB mounting brace, thus attaching the light pipe sub-assembly to both the PCB and the PCB mounting brace and compressing the spring. A status indicator lens is snapped onto the light pipe sub-assembly, thus defining a PCB assembly.

Various aspects of this invention will become apparent to those skilled in the art from the following detailed description of the preferred embodiment, when read in light of the accompanying drawings.

### BRIEF DESCRIPTION OF THE DRAWINGS

Fig. 1 is a perspective view of a first embodiment of an illuminated insect repeller in accordance with this invention.
Fig. 2 is a front elevational view of the illuminated insect repeller shown in Fig. 1.
Fig. 3 is a bottom view of the illuminated insect repeller shown in Fig. 1.
Fig. 4 is a cross sectional view of the illuminated insect repeller taken along line 4 - 4 of Fig. 1.
Fig. 5 is a cross sectional view of the illuminated insect repeller taken along line 5 - 5 of Fig. 1.
Fig. 6 is a perspective view of the battery housing shown in Figs. 4 and 5.
Fig. 7 is an alternate perspective view of the battery housing shown in Figs. 4 and 5.
Fig. 8 is a perspective view of a first side of the printed circuit board (PCB) shown in Figs. 4 and 5.
Fig. 9 is a partially exploded perspective view of a second side of the PCB shown in Figs. 4 and 5.
Fig. 10 is an exploded perspective view of the power button assembly shown in Figs. 1, 2, and 4.
Fig. 11 is a perspective view of the power button assembly shown prior to assembly to the PCB.
Fig. 12 is a front elevational view of the fluid reservoir in accordance with this invention.
Fig. 13 is a perspective view of a second embodiment of an insect repeller in accordance with the invention.
Fig. 14 is a is a cross sectional view of the insect repeller taken along line 14 - 14 of Fig. 13.
Fig. 15 is an alternate cross sectional view of the insect repeller taken along line 14 - 14 of Fig. 13.
Fig. 16 is a bottom view of the insect repeller shown in Fig. 13.
Fig. 17 is an exploded, perspective view of the heater module shown in Figs. 14 and 15.
Fig. 18 is a perspective view of the heater module shown in Fig. 17.
Fig. 19 is a cross sectional view of the heater module taken along line 19 - 19 of Fig. 18.
Fig. 20 is an exploded, perspective view of the cap assembly shown in Figs. 13 through 15.
Fig. 21 is a perspective view of the cap assembly shown in Fig. 20.
Fig. 22 is a cross sectional view of the cap assembly taken along line 22 - 22 of Fig. 21.
Fig. 23 is a perspective view of the main PCB assembly shown in Figs. 14 and 15.
Fig. 24 is an alternate perspective view of the main PCB assembly shown in Fig. 23.
Fig. 25 is an exploded, perspective view of the main PCB assembly shown in Figs. 23 and 24.
Fig. 26 is a perspective view of the base assembly shown in Figs. 14 and 15.
Fig. 27 is an exploded, perspective view of the base assembly shown in Fig. 26 showing an alternate embodiment of the battery and battery cradle.
Fig. 28 is a cross sectional view of the base assembly taken along line 28 - 28 of Fig. 26 showing the alternate embodiment of the battery and battery cradle.

### DETAILED DESCRIPTION OF THE PREFERRED EMBODIMENT

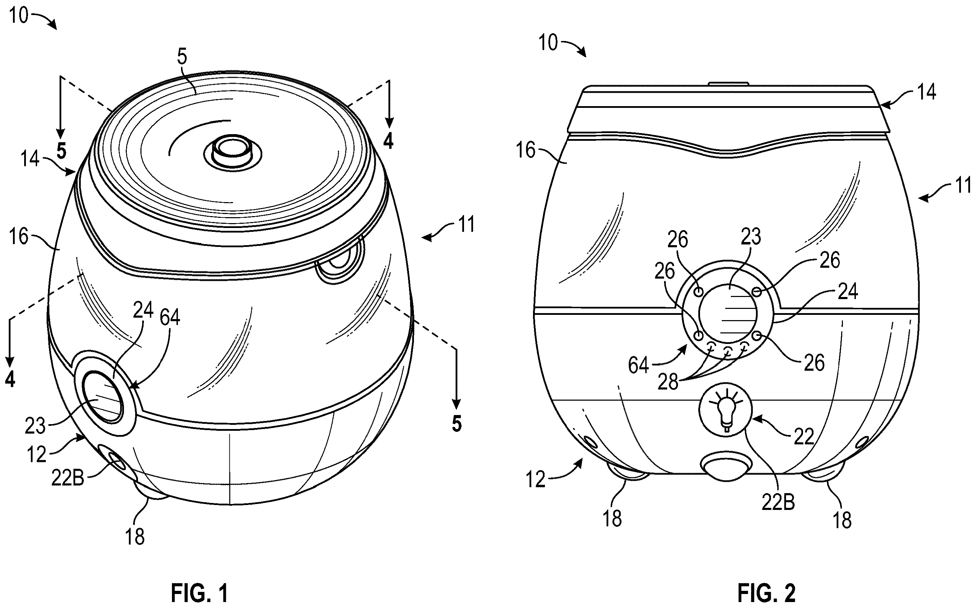

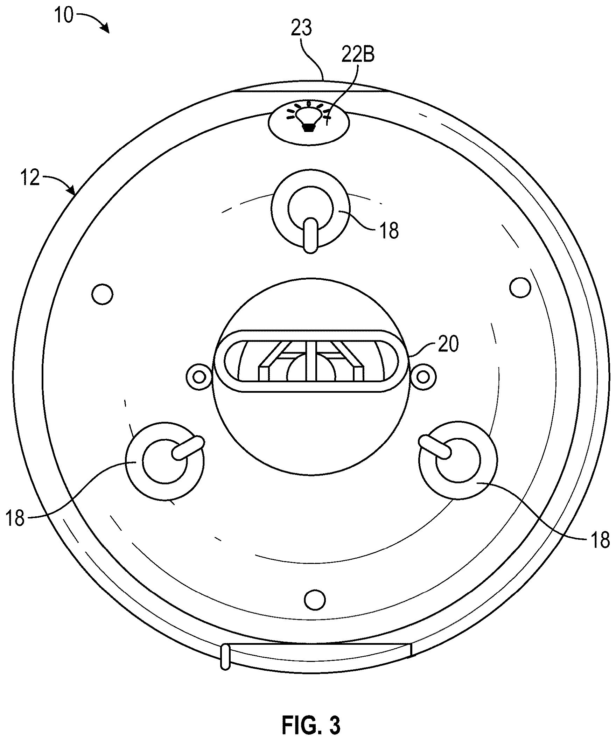

Referring now to the drawings, there is illustrated in Figs. 1 through 12, a first embodiment of an illuminated insect repeller 10. The illustrated illuminated insect repeller 10 includes a repeller housing 11 having a base 12, a cover assembly 14 and a main body assembly 13. The main body assembly 132 includes a main body portion 13A and an illumination panel 16 which defines a circumferentially extending side wall of the illuminated insect repeller 10. A lower surface of the base 12 includes a plurality of support feet 18. In the illustrated embodiment, the base 12 includes three support feet 18. However, it will be understood that the base 12 may have more than three support feet 18. In the illustrated embodiment, a lower surface of the base 12 also includes a vent and drain opening 20, best shown in Fig. 3. Each component of the repeller housing 11 may be formed from any desired rigid plastic material, including but not limited to polypropylene, glass filled polypropylene, polycarbonate, and blended polymers.

The base 12 further includes a light switch assembly 22, having a light switch 22A and a button 22B, wherein the button 22B is visible in Figs. 1 through 3. The base 12 additionally includes a status indicator lens 24, portions of which (see the circles 26) are selectively illuminated, as explained below, to indicate whether power and heat are operational in the illuminated insect repeller 10, and other portions of which (see the circles 28) are selectively illuminated to indicate a battery charge level. An on/off power button 23 is mounted within the status indicator lens 24 and is configured to engage the on/off power switch 52, described below.

The cover assembly 14 includes a first or upper portion 15A, and a second or lower portion 15B. The upper portion 15A includes a centrally formed opening through which a generally cylindrical heater chimney 30 of a heater assembly 31 extends. A plurality of magnets 2 are mounted, such as by press-fit, into recesses in an inside surface of the lower portion 15B.

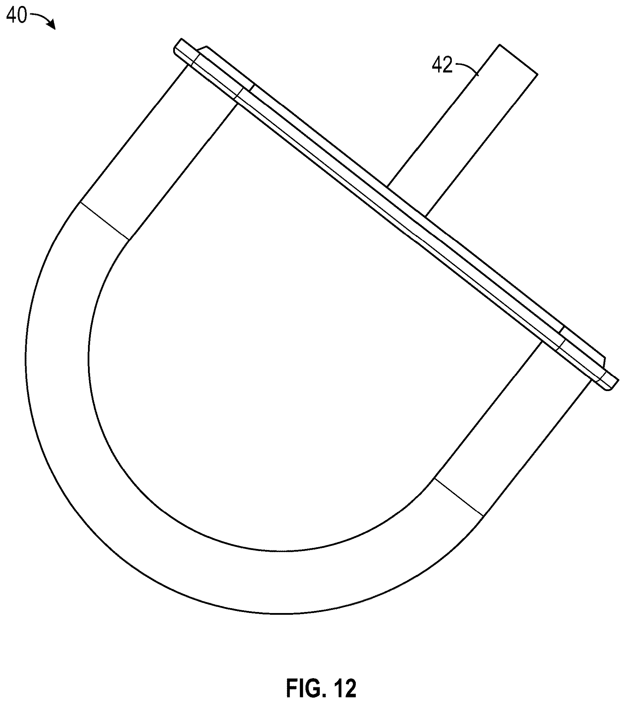

A reservoir holder 34 is centrally formed in the base 12 and is configured to hold a fluid reservoir 40, as shown in Fig. 12. The fluid reservoir 40 may be filled with insect repellant material and includes a wick 42 that extends into the interior of the fluid reservoir 40. When the fluid reservoir 40 is seated within the reservoir holder 34, the wick 42 extends outwardly into the heater 7. Liquid volatiles contained within the fluid reservoir 40 are conducted by capillary action out of the fluid reservoir 40 and to the distal end of the wick 42. The wick 42 is heated within the heater 7, causing the liquid volatiles to evaporate. This process continues until the reservoir is empty.

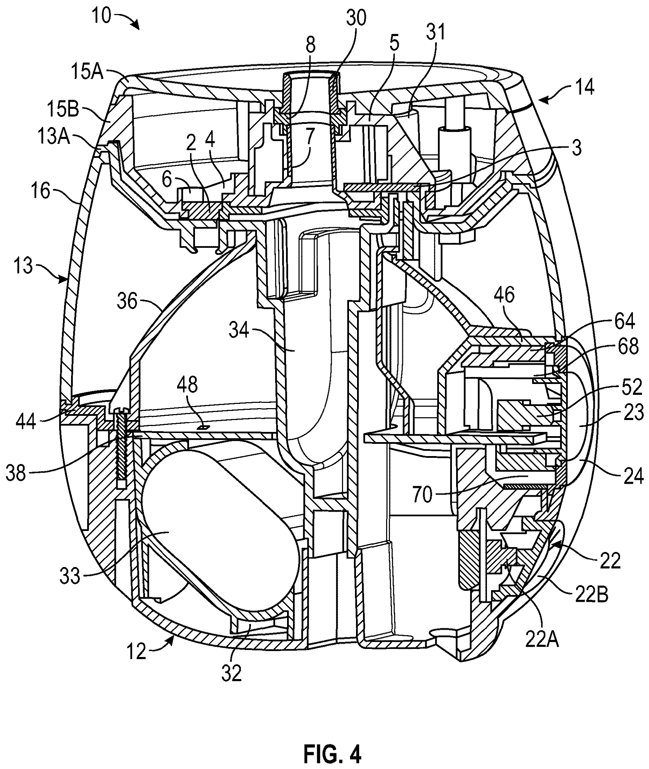

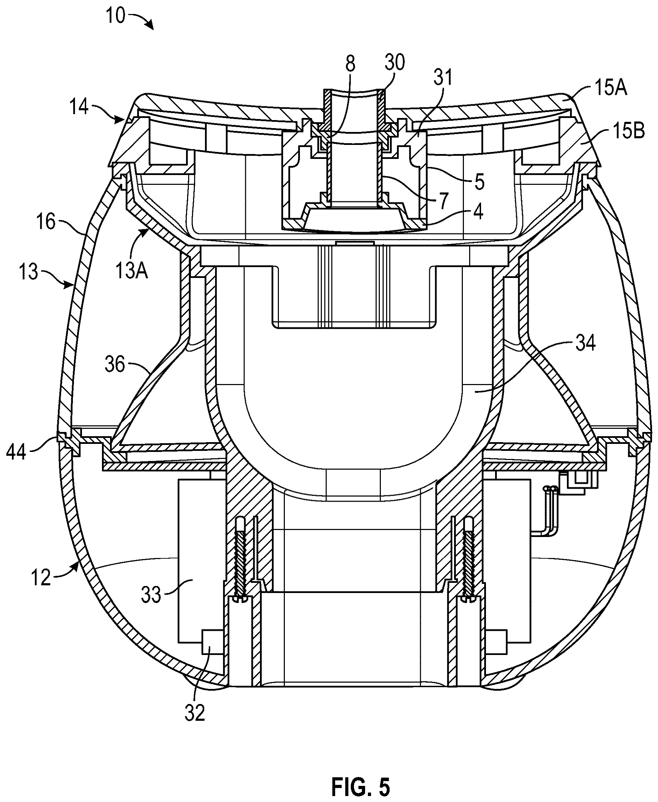

The heater assembly 31, best shown in Figs. 4 and 5, includes a heater PCB 3 mounted between a first portion or heater base 4 and a second portion or heater housing 5, wherein the heater base 4 and the heater housing 5 are attached by any desired method, including but not limited to mechanical fasteners and a snap-fit connection. The heater base 4 and the heater housing 5 are formed from any desired rigid, heat resistant plastic including but not limited to glass-filled nylon and other heat resistant plastic material. The heater base 4 includes three bosses or legs 6 extending outwardly from a lower surface of the heater base 4 (downwardly when viewing Fig. 4). The legs 6 engage the magnets 2 and ensure that the magnets 2 remain set in the recesses in the inside surface of the lower portion 15B.

A generally cylindrical heater 7 is electrically connected to the heater PCB 3 via electrical connectors (not shown). A cylindrical, resilient heater seal 8 is press fit through an opening in the heater housing 5 and circumferentially around a first end (the upper end when viewing Figs. 4 and 5) of the heater 7. As shown in Figs. 4 and 5, the heater assembly 31 is mounted within the lower portion 15B of the cover assembly 14. The heater seal 8 may be formed from any desired flexible material, including but not limited to, rubber, and other desired heat resistant elastomers that are compatible with the liquid volatiles.

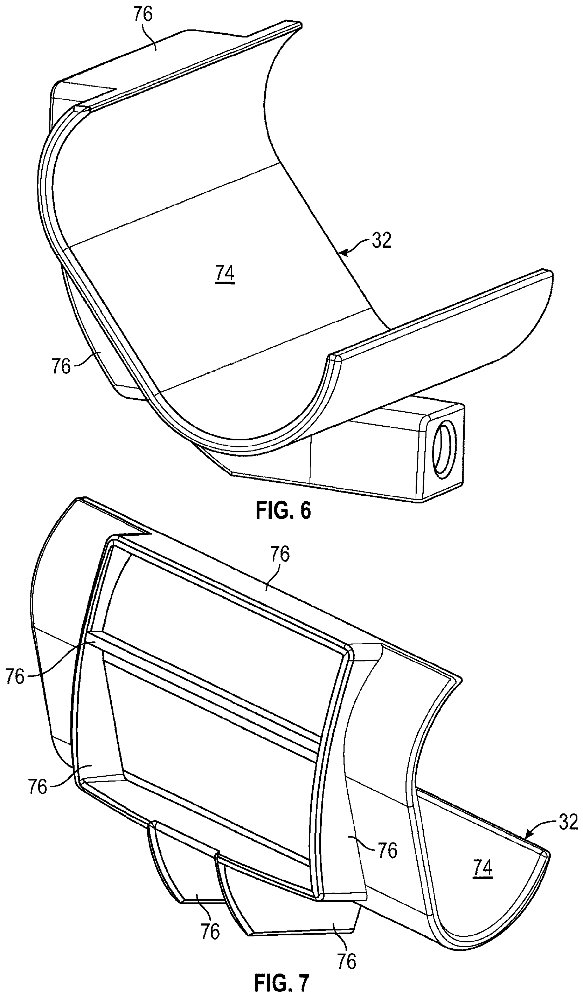

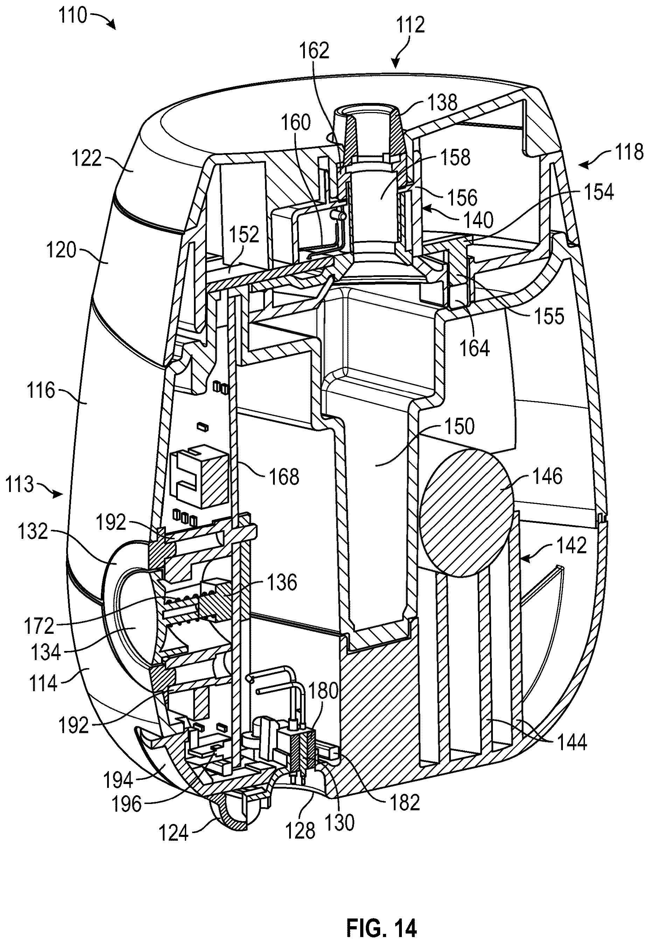

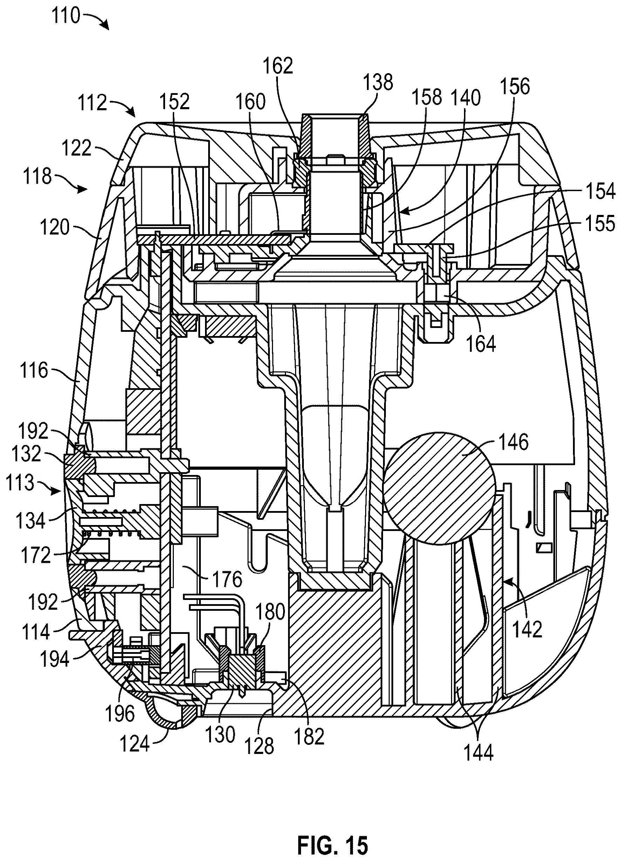

The base 12 includes a battery housing 32, configured for two batteries, such as for example, two rechargeable lithium-ion batteries 33. Alternatively, the battery housing may be configured for one battery, or other source of electrical power. As shown in Figs. 6 and 7, the battery housing 32 includes a cradle having generally C-shaped battery mounting surface 74, and an array of strengthening support ribs 76. Advantageously, the strengthening support ribs 76 provide sufficient strength and rigidity to the battery housing 32 such that the battery housing will survive accidental dropping during use with little or no damage, and such that some embodiments, including the embodiment shown in Figs. 14 and 15, the insect repeller may be manufactured without a conventional foam pad wrapped around the battery.

A mounting ring 44 includes a housing 46 for a power button assembly 64 and is mounted between the base 12 and the illumination panel 16.

A light diffusor panel 36 is mounted to the mounting ring 44 around the reservoir holder 34 and between the illumination panel 16 and a PCB 38. The PCB 38 is placed on mounting posts on an upwardly facing surface of the of the base 12 and the mounting ring 44 is place on an upwardly facing surface of the PCB 38. The light diffusor panel 36 is placed on an upwardly facing surface of the mounting ring 44. The light diffusor panel 36, the mounting ring 44, and the PCB 38 may then be attached together with fasteners, such as screws. The illumination panel 16 may be a translucent panel, a colored panel, or a clear panel.

The illuminated insect repeller 10 uses an electric heating element within the heater assembly 31 to volatize an insect repelling active ingredient, such as for example metofluthrin or similar compounds, to create an area around the illuminated insect repeller 10 having an insect-reduced or insect-free zone.

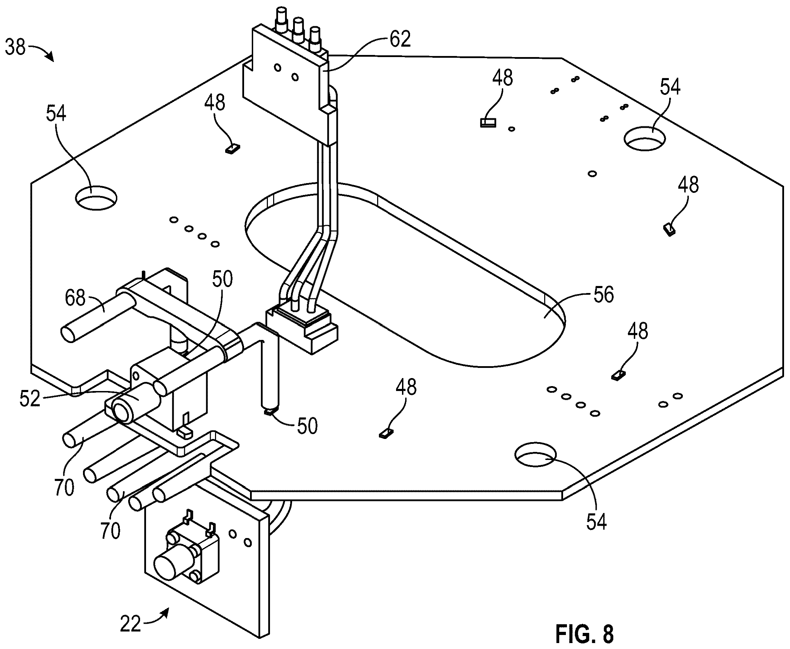

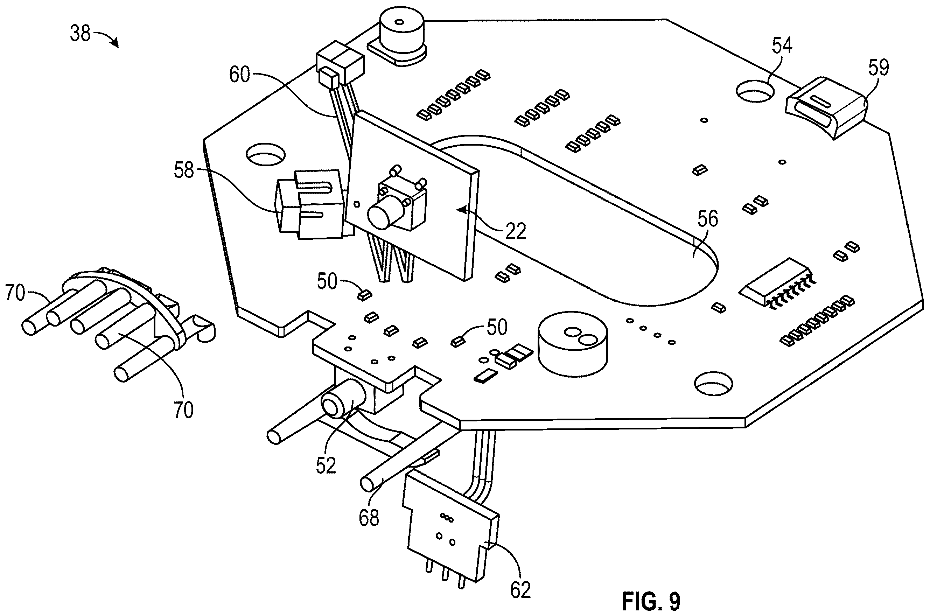

Referring now to Figs. 8 and 9, the PCB 38 is shown and includes six medium power LEDs 48 mounted to a first or upper side of the PCB 38 for illumination effect. Alternatively, the quality and power level of the LEDs may be varied depending on the light intensity, light color, and light effect desired. For example, LED lights may be provided to provide effects such as strobe, pulse, color change, varying light intensity, and other light effects.

LEDs 50 are provided on the PCB 38 for illuminating the power and heat indicator lights 26 and the battery charge level indicator lights 28 within the status indicator lens 24. In the illustrated embodiment, two LEDs 50 are mounted to the upper side of the PCB 38, as shown in Fig. 8, and five LEDs 50 are mounted to a second or lower side of the PCB 38, as shown in Fig. 9. An on/off power switch 52 is provided for moving the illuminated insect repeller 10 between on and off settings, portions of which (see the power and heat indicator lights 26) are selectively illuminated, as explained below, to indicate whether power and heat are operational in the illuminated insect repeller 10, and other portions of which (see the battery charge level indicator lights 28) are selectively illuminated to indicate a battery charge level. The PCB 38 includes mounting holes 54 for receiving attachment members, such as screws or bolts, an opening 56 configured to receive the fluid reservoir 40 and to provide drainage for the illuminated insect repeller 10, and a flying lead 62 for connection to the heater PCB 3 of the heater assembly 31.

As shown in Fig. 9, a second or lower side of the PCB 38 includes a battery connector 58, a USB-C connector port 59, and a flying lead 60 for connection to the light switch assembly 22.

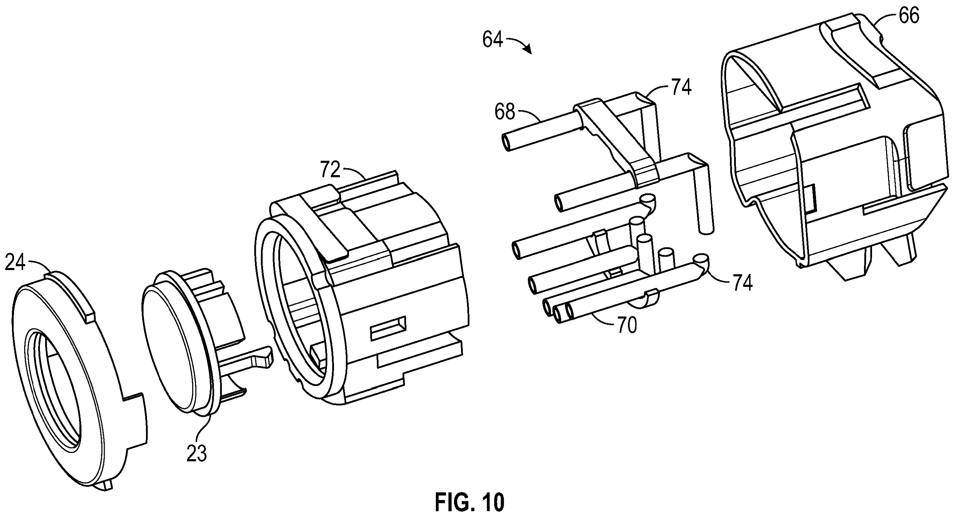

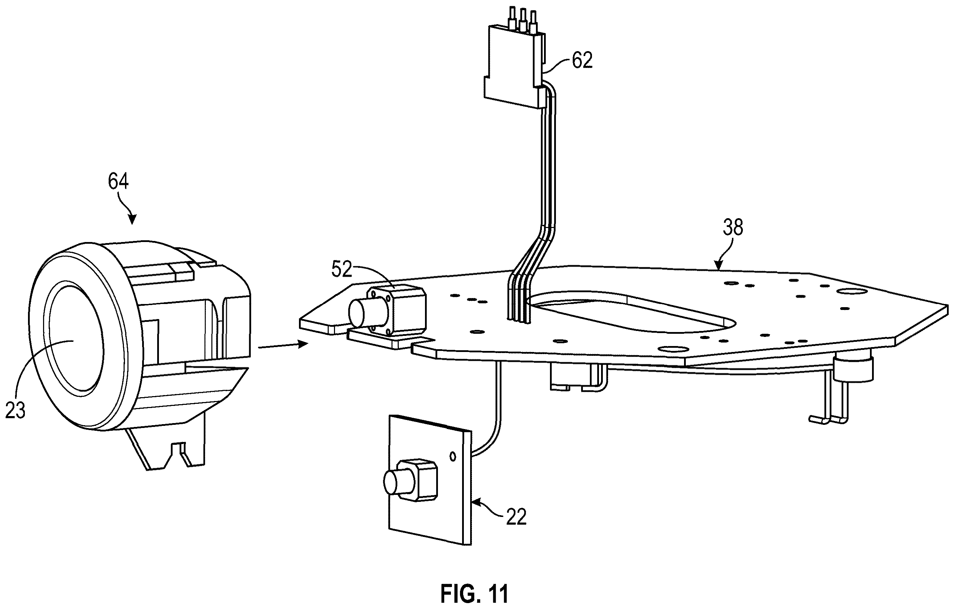

Referring now to Figs. 10 and 11, the power button assembly is shown at 64. The power button assembly 64 includes a light pipe shroud 66, a pair of upper light pipes 68, five lower light pipes 70, and a light pipe housing 72 within which the light pipes 68 and 70 are mounted. The light pipe housing 72 is mounted within the light pipe shroud 66. The on/off power button 23 is mounted within the light pipe housing 72, and the status indicator lens 24 is mounted to an outside surface of the light pipe housing 72. The power button assembly 64 is attached to the PCB 38 about the on/off power switch 52 and is configured such that the on/off power button 23 engages the on/off power switch 52. As shown in Fig. 11, the power button assembly 64 may be attached by sliding the power button assembly 64 onto the PCB 38.

The illumination panel 16 projects light of a user-selected intensity. The light diffusor panel 36 receives light radiating from a plurality of light sources, such as the LEDs 48, and transmits a uniform, or generally homogenized, light spectrum to the illumination panel 16. The light diffusor panel 36 generates a spectral light wave output to the illumination panel 16 such that light output of the illumination panel 16 is generally the same, or uniform, along an entire surface of the illumination panel 16. Because the components of the illuminated insect repeller 10, other than the illumination panel 16 and the light diffusor panel 36 are opaque, light output through the light diffusor panel 36 to the illumination panel 36 clean and uniform to the user of the illuminated insect repeller 10. The light diffusor panel 36 may be a translucent panel formed from a polymer or glass material. In one embodiment, the polymer may be, for example, formed from a polycarbonate, polyethylene, or polyolefin material, although other materials may be used.

The light spectrum may contain any desired range or ranges of light wavelengths. In one embodiment, the light wavelengths are generally within the visible light spectrum. In yet another embodiment, the light wavelengths are characterized by a light temperature factor, such as light defined on a Kelvin scale for color intensity characterization. It is known that many species of mosquitoes are attracted to light, and those species may be attracted to products that include lights. There are several mosquito traps that employ lights to increase their catch efficacy by relying on the light spectrum to attract insects. Not all mosquito species respond in the same way however, and the impact of light on mosquito behavior varies by mosquito species. Light is also not as attractive as carbon dioxide (CO₂) as a host-seeking cue. The combination of both light and CO₂, however, commonly results in higher trap catch numbers than either variable alone. Therefore, assuming that these devices are operated near humans exhaling CO₂, the addition of light may increase the attraction of local mosquitoes.

With this relationship better understood, it is desirable to minimize the impact that adding or using lights has on attracting insects in the context of an insect repeller. Certain wavelengths of light and/or colors of lights have been investigated for their relative attractiveness to various mosquito species. Lights in the green color range and in the blue - purple range have been shown to be relatively attractive to certain species of mosquitoes. The UV range of wavelengths has also been shown to be widely attractive to various insects, not just mosquitoes. In contrast, colors in the red - yellow range do not result in significant increases in mosquito trap catches, indicating that these colors would not represent a significant attractive element when added to a device. Specifically, colors below about 3,000 Kelvin generally minimize this impact. In one embodiment, the color temperature is below 2700 Kelvin.

Intensity of light may also be considered when attempting to minimize the impact of light on mosquito attraction. It has been shown that increasing light intensity causes a linear increase in the trap catches of associated mosquito traps that rely on attracting insects. In one embodiment of the illuminated insect repeller 10, light intensity may be user-selectable between a range of 100% or full light emission capacity based on the light source output, to a low intensity range, such as 20% of maximum illumination intensity.

The illumination light sources are mounted on the PCB 38 over a dispersed area to transmit illumination light evenly to the diffusion panel without creating areas of noticeable changing intensities of light. To minimize impact to the lighting effect and prevent shadowing due to electronics in the illumination area, the PCB 38 is oriented horizontally, or generally parallel to the cover assembly 14. Due to the horizontal orientation of the PCB 38 and surface mounted light sources, such as the LEDs 50 provided on the PCB 38 for illuminating for the power and heat indicator lights 26 and the battery charge level indicator lights 28, the light pipes 68 and 70 are used to transition the indicator lighting to the status indicator lens 24 and provide feedback on functionality to the consumer. The light pipes 68 and 70 include a light input end that is positioned proximate to status light sources, i.e., the LEDs 50, typically in a one-to-one relationship. The light pipes 68 and 70 further include an output that projects a status light signal to the status indicator lens 24 to alert a user of the operational status of the illuminated insect repeller 10.

Alternatively, the PCB 38 may include one or more LEDs 48, or other sources of light, mounted to a second or lower side of the PCB 38. The PCB 38 may be mounted in other locations within the 10, such as closer to the cover assembly 14 and such that light from the LEDs 48 mounted on the lower side of the PCB 38 is directed downwardly for illumination effect. The PCB 38 may also include LEDs 48 mounted to both the lower and upper sides thereof. Further, the quality and power level of the LEDs may be varied depending on the light intensity, light color, and light effect desired. For example, LED lights may be provided to provide effects such as strobe, pulse, color change, varying light intensity, and other light effects.

the PCB 38 may have other shapes, configurations, locations, and orientations within the illuminated insect repeller 10. For example, the PCB may be cylindrical, conical, or frusto-conical in shape and be positioned around the reservoir holder 34 within the base 12.

In order to transition from the horizontally mounted LEDs 50 to a vertical indicator output in the status indicator lens 24, the light pipes 68 and 70 are configured to create a bent or angled pathway. In one embodiment, the light pipes 68 and 70 may be configured to project light through a 90 degree angle, though any other angle may be contemplated within the scope of the invention. At a point generally coincident with the intersection points of the horizontal and vertical pathways, a section 74 of each light pipe 68 and 70 is angled at a bisecting orientation to the vertical and horizontal light pathways. In one embodiment, the pathways intersect at a right angle and the bisecting orientation is approximately 45 degrees, as shown in Fig. 10. The angled section is conditioned, such as by polishing or mirror coating, to reflect the incoming light rays to the output associated with the status indicator lens 24. In order to maximize the light transmission and minimize light bleeding from other sources, an opaque housing shields and separates each light source from other light sources, such as the illumination lights 48 or other status indicator lights, such as the LEDs 50. In an alternative embodiment, the light pipes 68 and 70 may each include a light gasket or light pipe shield that extends from the input end of the light pipe and surrounds each of the light to pipe interfaces. The light pipes 68 and 70 may further have a coating or outer surface that prevents light from entering at points along the light pipes 68 and 70.

It will be understood that the power button assembly 64 may be provided without the light pipes 68 and 70, such that light is reflected using only polished inner surfaces of the light pipe shroud 66 and the light pipe.

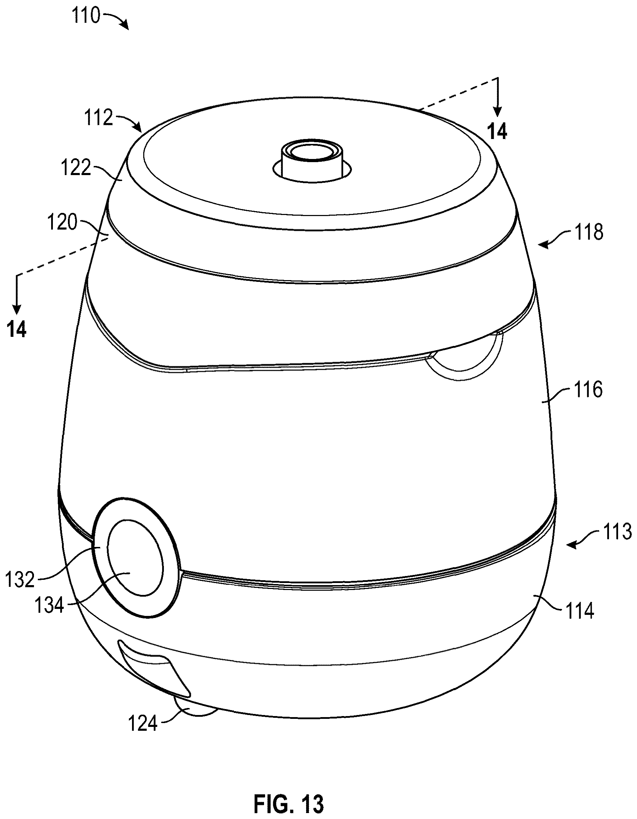

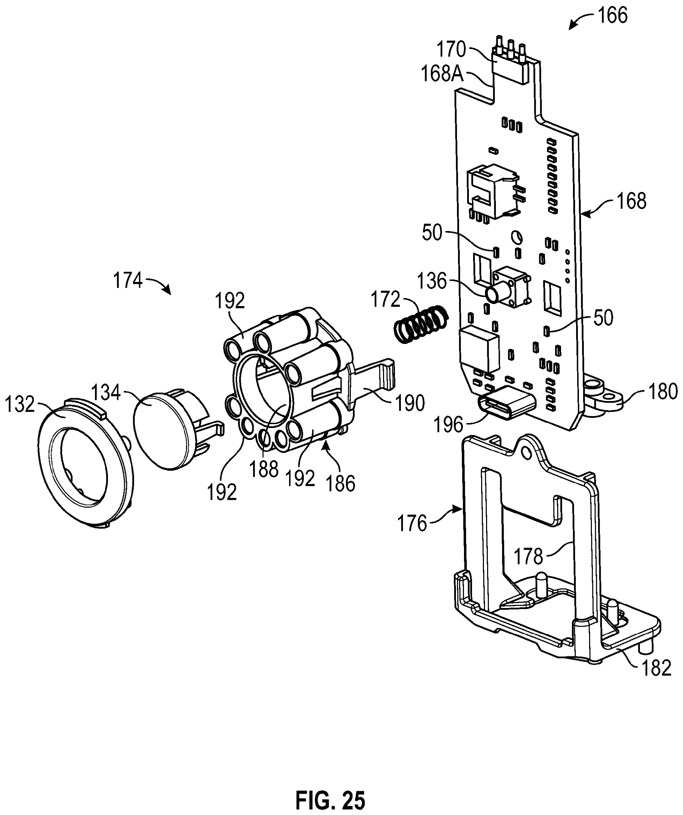

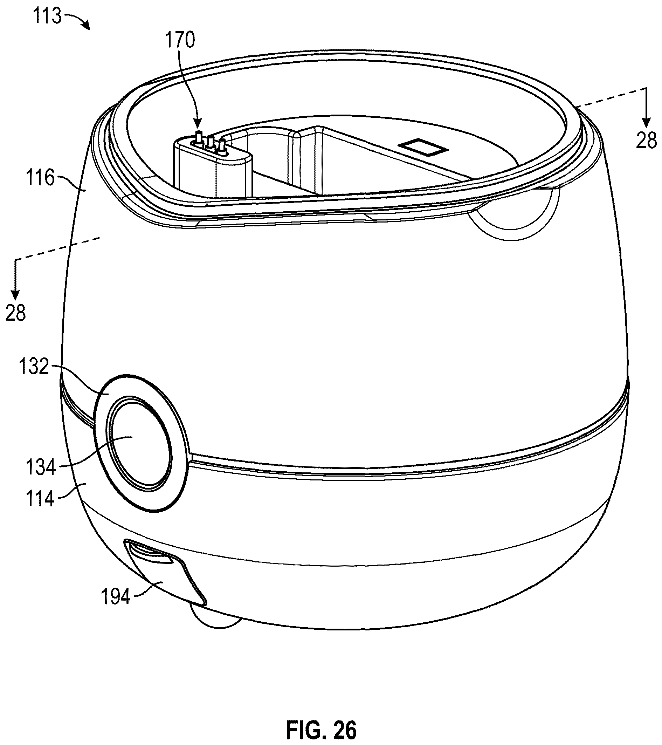

Referring now to Figs. 13 through 28, there is illustrated a second embodiment of the insect repeller 110. The illustrated insect repeller is similar to the first embodiment of the insect repeller, but may not include the illumination panel 16 of the illuminated insect repeller 10. It will be understood however, that the insect repeller 110 may, if desired, include an illumination panel similar to the illumination panel 16. The illustrated embodiment of the insect repeller 110 includes a repeller housing 112 includes a housing base assembly 113 having a base 114 and a main housing portion 116, and a two-part cap assembly 118 having a cap base 120 and a cap top 122. Each component of the repeller housing 112 may be formed from any desired rigid plastic material, including but not limited to polypropylene, glass filled polypropylene, polycarbonate, and blended polymers.

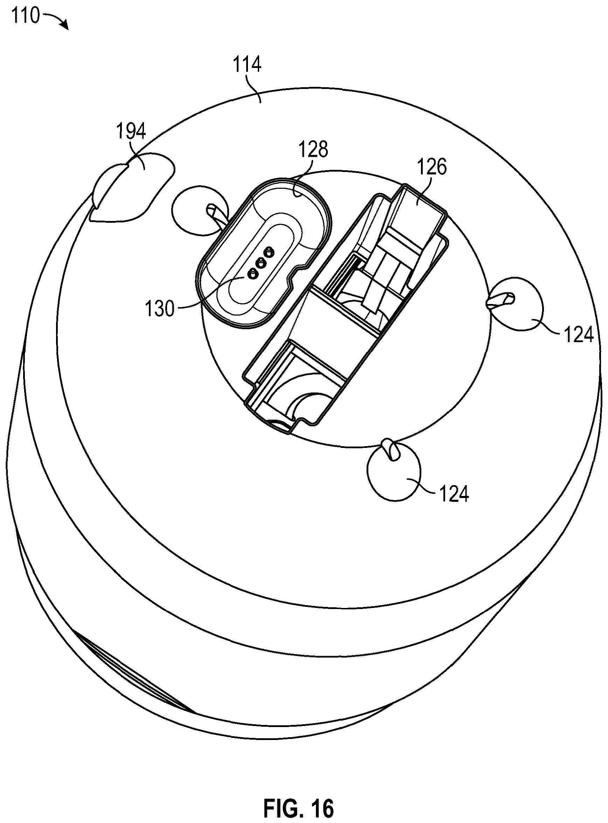

A lower surface of the base 114 includes a plurality of support feet 124. In the illustrated embodiment, the base 114 includes three support feet 124. However, it will be understood that the base 114 may have more than three support feet 124. In the illustrated embodiment, a lower surface of the base 114 also includes a vent and drain opening 126, and an electrical connection port 128 having pogo pins 130 therein and configured for connection to an associated docking station (not shown), as best shown in Fig. 16.

The base 114 further includes a status indicator lens 132, portions of which are selectively illuminated, as explained below, to indicate whether power and heat are operational in the insect repeller 110, and other portions of which are selectively illuminated to indicate a battery charge level. An on/off power button 134 is mounted within the status indicator lens 132 and is configured to engage an on/off power switch 136, described below.

The cap top 122 of the cap assembly 118 includes a centrally formed opening through which a generally cylindrical heater chimney 138 of a heater module 140 extends.

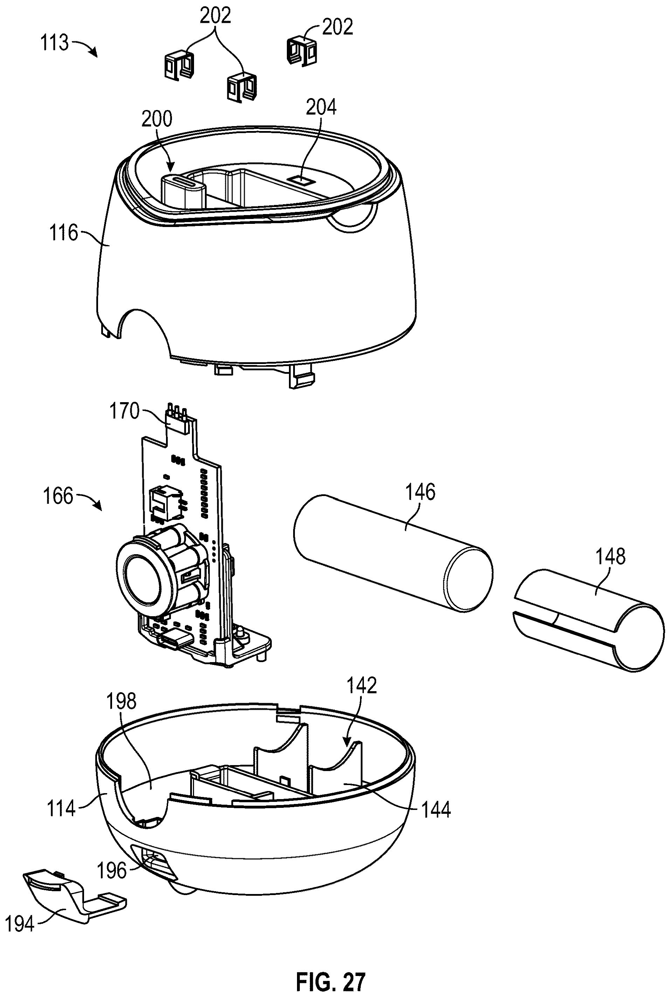

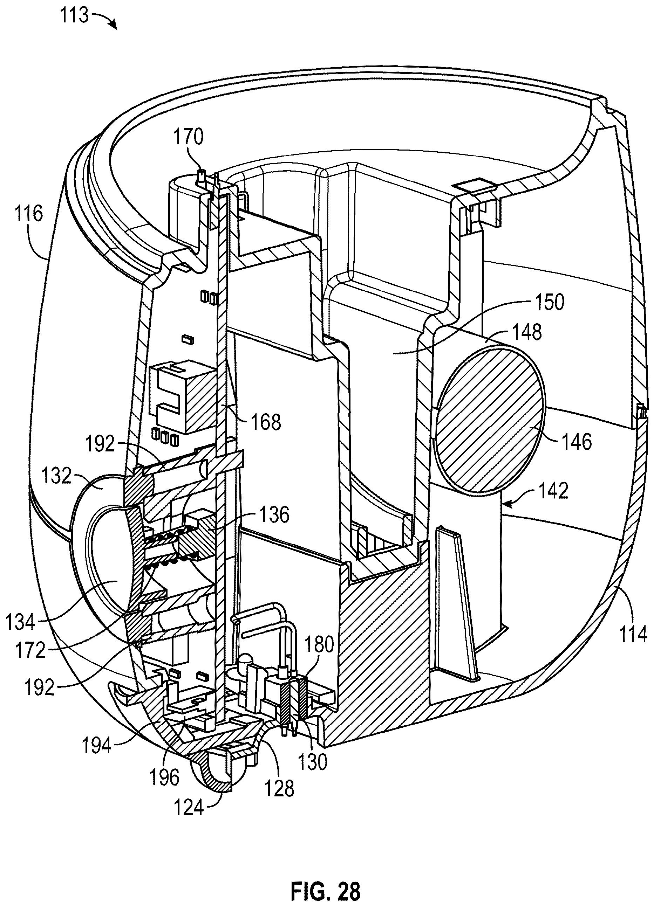

The base 114 includes a battery cradle 142 having a plurality of strengthening support ribs 144, and configured to support a battery 146, such as for example, a rechargeable lithium-ion battery. Although in the illustrated embodiment one battery 146 is shown, it will be understood that the base 114 and the battery cradle 142 may be sized and configured to support batteries (not shown). The insect repeller 110 may be manufactured without a conventional foam pad wrapped around the battery 146, as shown in Figs. 14 and 15. In an alternate embodiment of the insect repeller shown in Figs. 27 and 28, a foam pad 148 is provided and may be wrapped around the battery 146.

A reservoir holder 150 is centrally formed in the main housing portion 116 and is configured to hold the fluid reservoir 40, as shown in Fig. 12. The fluid reservoir 40, shown in Fig. 12, may be filled with insect repellant material and includes a wick 42 that extends into the interior of the fluid reservoir 40. When the fluid reservoir 40 is seated within the reservoir holder 150, the wick 42 extends outwardly into a heater 158. Liquid volatiles contained within the fluid reservoir 40 are conducted by capillary action out of the fluid reservoir 40 and to the distal end of the wick 42. The wick 42 is heated within the heater 158, causing the liquid volatiles to evaporate. This process continues until the reservoir is empty.

The heater module 140, best shown in Figs. 16 through 18 includes a heater PCB 152 mounted between a first portion or heater base 154 and a second portion or heater housing 156, wherein the heater base 154 and the heater housing 156 are attached via a snap-fit connection.

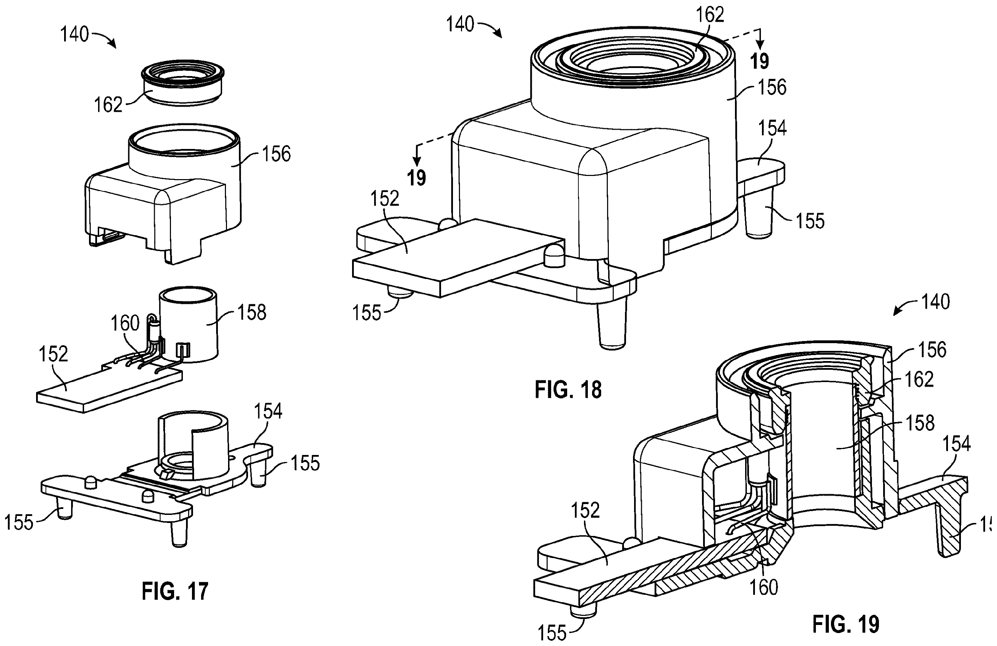

The heater base 154 and the heater housing 156 are formed from any desired rigid, heat resistant plastic including, but not limited to, glass-filled nylon and other heat resistant plastic material. The heater base 154 includes three bosses or legs 155 extending outwardly from a lower surface of the heater base 154 (downwardly when viewing Figs. 17 through 19). The legs 155 engage magnets 164, described below, and ensure that the magnets 164 remain set in the recesses in an inside surface of the cap base 120.

The heater PCB 152 includes the generally cylindrical heater 158 electrically connected to the heater PCB 152 via electrical connectors 160. A cylindrical, resilient heater seal 162 is press fit through an opening in the heater housing 156 and circumferentially around a first end (the upper end when viewing Figs. 17 through 19) of the heater 158. As shown in Figs. 13 and 14, the heater module 140 is mounted within the cap base 120 of the cap assembly 118.

During assembly of the heater module 140, the heater 158 is electrically connected to the heater PCB 152 via electrical connectors 160 to define a heater PCB assembly 153. The heater PCB assembly 153 is placed on the heater base 154. The heater housing 156 is then placed on to the heater PCB assembly 153 and attached to the heater base 154 via a snap-fit connection. The heater seal 162 is then press fit through an opening in the heater housing 156 and circumferentially around a first end (the upper end when viewing Figs. 17 through 19) of the heater 158. The heater seal 162 may be formed from any desired flexible material, including but not limited to, rubber, and other desired heat resistant elastomers that are compatible with the liquid volatiles.

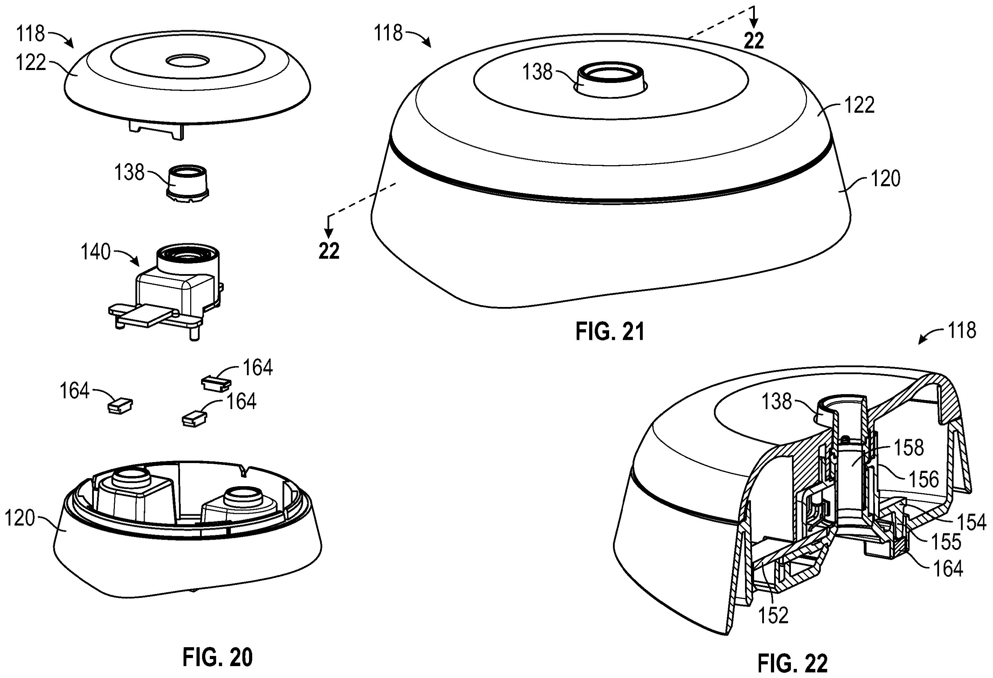

The assembled heater module 140 may then be mounted within the cap assembly 118. As shown in Figs. 20 through 22, a plurality of magnets 164 are mounted, such as by press-fit, into recesses in an inside surface of the cap base 120. The heater module 140 is then mounted centrally within the cap base 120 such that the legs 155 engage the magnets 164. The heater chimney 138 is then attached to the heater seal 162 and the cap top 122 is then attached to the cap base 120 via a snap-fit connection, such that the heater chimney 138 extends outwardly of the centrally formed opening in the cap top 122.

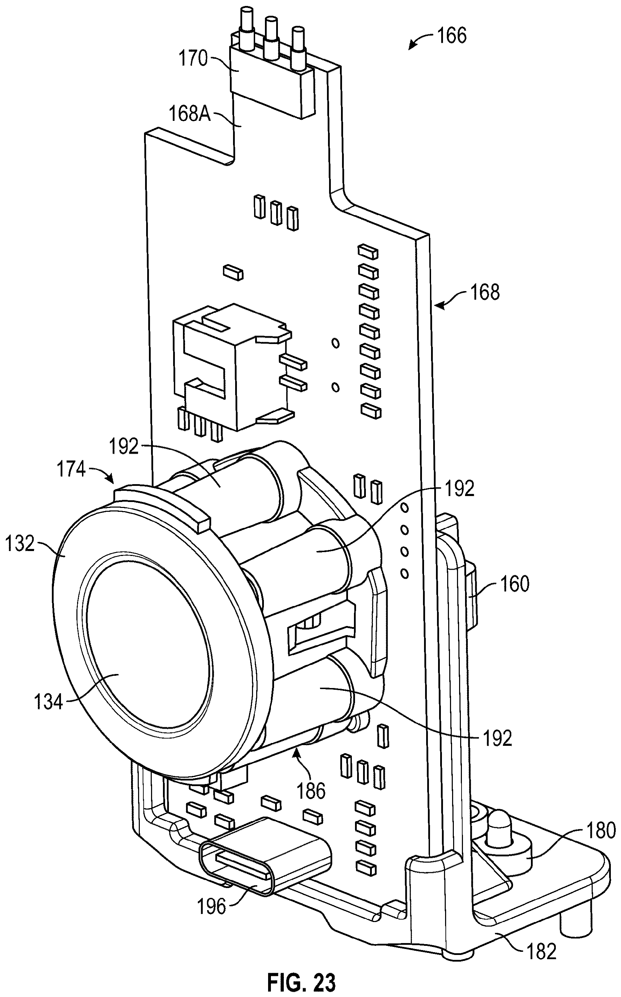

A main PCB assembly 166 and a method of assembling the main PCB assembly 166 are shown in Figs. 23 through 25. The illustrated embodiment of the main PCB assembly 166 includes a conventional PCB 168, oriented vertically, or substantially parallel with a longitudinal axis of the insect repeller 110. Pogo pins 170, or other springloaded contact probes, are mounted to a first end 168A of the PCB 168 (the upper end when viewing Figs. 23 through 25). A first side of the PCB 168 may include a microcontroller, other required functional electronic components, and the on/off power switch 136 having an outwardly extending post electrically mounted to the PCB 168 and necessary to operate the insect repeller 110. Illumination light sources, such as the LEDs 50 are provided on the PCB 168 for illuminating the power and heat indicator lights (not shown) and the battery charge level indicator lights (not shown) within the status indicator lens 132. For example, the insect repeller 110 may be provided with 7 LEDs 50 that include four chasing LEDs 50; i.e., LEDs 50 that chase during warm-up of the heater module 140, and that turn solid when the insect repeller 110 is on and warmed-up, and three LEDs 50 that indicate battery charging and the battery status.

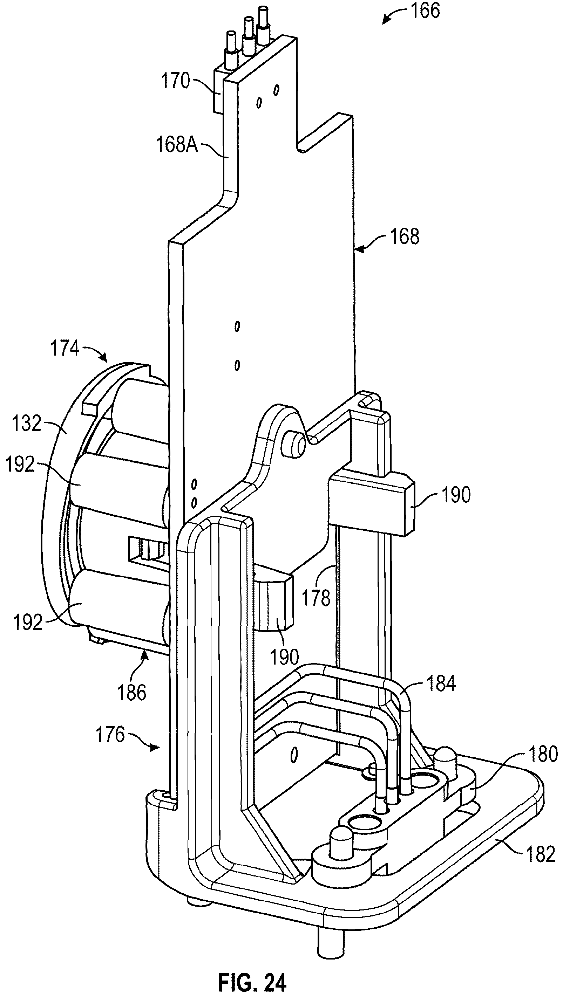

The main PCB assembly 166 further includes the status indicator lens 132, the on/off power button 134, a spring 172 that extends between the post of the on/off power switch 136, a light pipe sub-assembly 174, and a PCB mounting frame or brace 176 having a PCB access opening 178 formed therethrough. The PCB mounting brace 176 includes an electrical connector 180 mounted to a base 182 thereof. The electrical connector 180 includes the plurality of pogo pins 130 extending outwardly (downwardly when viewing Fig. 24, and best shown in Fig. 28) from a first surface of the electrical connector 180 (downwardly facing surface when viewing Fig. 24), and pogo pin electrical leads 184 extending from a second surface (upwardly facing surface when viewing Fig. 24) of the electrical connector 180 on the base 182 of the mounting brace 176, through the PCB access opening 178, to a second side of the PCB 168.

The light pipe sub-assembly 174 includes a light pipe housing 186 having a button opening 188 centrally formed therein, attachment arms 190 extending outward thereof, and a plurality of light pipes 192. In the illustrated embodiment eight light pipes 192 are shown, however it will be understood that the light pipe sub-assembly 174 may include any desired number of light pipes 192. The light pipe sub-assembly 174 is substantially similar to the light pipes 68 and 70 described above in reference to the first embodiment of the illuminated insect repeller 10.

The method of assembling the main PCB assembly 166, as shown in Figs. 23 through 25, includes mounting to a first side of the PCB 168 the required components necessary to operate the insect repeller 110, including for example, the microcontroller, other required functional electronic components, and the on/off power switch 136 having an outwardly extending post. The electrical connector 180 is mounted to the base 182 of the PCB mounting brace 176. The PCB 168 is mounted into a groove formed in the PCB mounting brace 176. Pogo pin electrical leads 184 from the electrical connector 180 on the base 182 of the mounting brace 176 are connected to the second side of the PCB 168 through the PCB access opening 178.

The pogo pins 170 are placed onto posts and posts are heat staked to lock the pogo pins 170 in place at the first end 168A of the PCB 168. The on/off power button 134 is then snapped into the button opening 188 of the light pipe housing 186. The spring 172 is attached to the post of the on/off power switch 136, and the attachment arms 190 of the light pipe sub-assembly 174 are snapped the through apertures in the PCB 168 to engage and snap onto the PCB mounting brace 176, thus attaching the light pipe sub-assembly 174 to both the PCB 168 and the PCB mounting brace 176 and compressing the spring 172. The status indicator lens 132 is then snapped onto the light pipe sub-assembly 174, thus defining the main PCB assembly 166.

Once the main PCB assembly 166 is assembled, the housing base assembly 113 may be assembled. To assemble the housing base assembly 113, a USB plug 194 is inserted into a USB-C connector port opening 196 in the housing base 114. The USB plug 194 may be formed from any desired resilient material, such as buna rubber and other elastomers. The assembled PCB assembly 166 is then plugged into a cavity 198 in the housing base 114. The pogo pins 130 of the electrical connector 180 of the PCB mounting brace 176 are then snapped into the electrical connection port 128 of housing base 114 to position the PCB assembly 166 within the repeller housing 112.

Advantageously, the pogo pins 130 of the electrical connector 180 are located and configured to dock, or be connected to, a docking station (not shown) as an alternate method to electrically charge the insect repeller 110, in addition to using the USB-C port opening 196.

The battery 146 is attached to the PCB assembly 166 and placed on ribs 144 formed in the housing base 114 and defining the battery cradle 142. The main housing portion 116 is then snapped onto the housing base 114 such that the pogo pins 170 extend through a slot in a pin port 200 formed in the main housing portion 116. A plurality of snap clips 202 are mounted in clip cavities 204 formed in the main housing portion 116. The cap assembly 118 is then mounted on the housing base assembly 113 such that the magnets 164 in the cap assembly 118 engage a surface of the snap clips 202 mounted in the housing base assembly 113.

The following numbered statements form part of the present disclosure:
Statement 1. An illuminated insect repeller comprising:
   a heater assembly;
   an electrical power source;
   a repeller power switch; and
   a power button assembly including a status indicator lens, a repeller power button, and at least one light pipe optically connected to the status indicator lens and configured to project light from a light source to the status indicator lens;
   wherein the repeller power switch is in electrical communication between the electrical power source and the heater assembly.
Statement 2. The illuminated insect repeller according to Statement 1, further including a printed circuit board (PCB), the PCB including the light source and configured to control operation of the heater assembly and generate a status signal from an output of the light source to the status indicator lens.
Statement 3. The illuminated insect repeller according to Statement 2, wherein the light source includes a plurality of first LEDs mounted to both sides of the PCB.
Statement 4. The illuminated insect repeller according to Statement 3, further including a housing configured to support the heater assembly, the electrical power source, and the PCB;
   wherein the housing includes a base, a cover, an illumination panel between the base and the cover, and a light diffuser panel mounted within the housing between the illumination panel the PCB; and
   wherein the PCB includes a plurality of second LEDs on a first side thereof, the first side of the PCB facing the light diffuser panel.
Statement 5. The illuminated insect repeller according to Statement 4, wherein the plurality of second LEDs is controlled by a light switch to selectively control output the plurality of second LEDs, the output being between an off state and an illumination state, the illumination state controllable between a 100 percent brightness state of the plurality of second LEDs and a 20 percent brightness state of the plurality of second LEDs.
Statement 6. The illuminated insect repeller according to Statement 5, wherein the PCB further includes an electrical connector extending outwardly of an opening in the base and configured for connection to a corresponding electrical connector in a docking station, the docking station configured to provide electrical power to the illuminated insect repeller.
Statement 7. The illuminated insect repeller according to Statement 3, further including a housing configured to support the heater assembly, the electrical power source, and the PCB;
   wherein the housing includes a base, a cover, an illumination panel between the base and the cover, and a light diffuser panel mounted within the housing between the illumination panel the PCB; and
   wherein the power button assembly further includes a plurality of light pipes optically connected to the status indicator lens and configured to project light from the plurality of first LEDs mounted to both sides of the PCB through an angle to the status indicator lens.
Statement 8. The illuminated insect repeller according to Statement 7, wherein the power button assembly includes a light pipe shroud, a plurality of upper light pipes, a plurality of lower light pipes, and a light pipe housing within which the light pipes are mounted; wherein the light pipe housing is mounted within the light pipe shroud; wherein the repeller power button is mounted within the light pipe housing, and the status indicator lens is mounted to an outside surface of the light pipe housing; wherein the power button assembly is attached to the PCB via a sliding connection between a slot formed in the light pipe shroud about the repeller power switch; and wherein the power button assembly is configured such that the repeller power button engages the repeller power switch.
Statement 9. The illuminated insect repeller according to Statement 8, wherein the illumination panel is in spectral light wave communication with the light diffusor panel; and wherein the light diffusor panel generates a spectral light wave output to the illumination panel such that light output of the illumination panel is generally the same along an entire surface of the illumination panel.
Statement 10. The illuminated insect repeller according to Statement 9, wherein the illumination panel is one of a translucent panel, a colored panel, and a clear panel.
Statement 11. The illuminated insect repeller according to Statement 7, wherein the base includes a battery housing configured to support at least one battery, and wherein the battery housing includes a cradle having a battery mounting surface, and an array of strengthening support ribs.
Statement 12. The illuminated insect repeller according to Statement 1, wherein the at least one light pipe is optically connected to the status indicator lens and configured to project light from a light source through an angle to the status indicator lens.
Statement 13.A printed circuit board (PCB) assembly for an insect repeller comprising:
   a PCB mounting brace having a base and a PCB access opening formed therethrough;
   a PCB having a first and a second side, the PCB having a microcontroller, one or more functional electronic components, and a power switch having an outwardly extending power switch post electrically mounted to the first side of the PCB and configured to operate the insect repeller, the PCB mounted within a groove formed in the PCB mounting brace;
   a plurality of first pogo pins mounted to a first end of the PCB;
   an electrical connector mounted to a base of the PCB mounting brace, the electrical connector having a plurality of second pogo pins and electrical leads extending from the electrical connector through the PCB access opening, to the second side of the PCB;
   a light pipe sub-assembly including a light pipe housing having a button opening centrally formed therein, attachment arms extending outward thereof, and a plurality of light pipes, the attachment arms extending through the PCB and into the PCB mounting brace, a power button mounted within the button opening, a status indicator lens attached to the light pipe sub-assembly, and a spring mounted between the power switch post and the power button.
Statement 14. The PCB assembly according to Statement 13, further including a plurality of light sources mounted to the first side of the PCB.
Statement 15. The PCB assembly according to Statement 14, wherein the light sources are LEDs.
Statement 16. The PCB assembly according to Statement 15, wherein the plurality of light pipes extends between the first side of the PCB and the status indicator lens, the plurality of light pipes configured to project light from the LEDs to the status indicator lens.
Statement 17. The PCB assembly according to Statement 16, wherein the PCB is mounted in an insect repeller housing, the insect repeller housing including a housing base assembly having a base and a main housing portion, and a two-part cap assembly having a cap base and a cap top.
Statement 18. The PCB assembly according to Statement 17, wherein the insect repeller housing further includes a heater assembly mounted in the cap assembly.
Statement 19. The PCB assembly according to Statement 18, wherein the heater assembly includes
   a heater PCB mounted between a heater base and a heater housing, wherein the heater base and the heater housing are attached via a snap-fit connection, and wherein the heater base includes three legs extending outwardly from a lower surface of the heater base;
   a cylindrical heater electrically connected to the heater PCB via electrical connectors;
   a cylindrical heater seal press mounted circumferentially around a first end of the heater; and
   a heater chimney attached to the heater seal opposite the heater, such that the heater chimney extends outwardly of a centrally formed opening in the cap top.
Statement 20. The PCB assembly according to Statement 19, wherein the base includes a battery housing configured to support at least one battery, and wherein the battery housing includes a cradle having a battery mounting surface, and an array of strengthening support ribs.
Statement 21. The PCB assembly according to Statement 20, wherein the second pogo pins extend outwardly of an opening in the base and are configured for connection to a corresponding electrical connector in a docking station, the docking station configured to provide electrical power to an insect repeller within which the PCB assembly is mounted.
Statement 22.A method of assembling a printed circuit board (PCB) assembly for an insect repeller, the method comprising:
   mounting to a first side of a PCB electrical components necessary to operate the insect repeller, including at least one of a microcontroller, one or more functional electronic components, a power button having an outwardly extending power switch post electrically mounted to the first side of the PCB and configured to operate the insect repeller;
   placing a plurality of first pogo pins onto posts and heat staking the posts to lock the plurality of pogo pins in place at a first end of the PCB;
   mounting an electrical connector to a base of a PCB mounting brace, the electrical connector having a plurality of second pogo pins and pogo pin electrical leads extending from the electrical connector;
   mounting the PCB into a groove formed in the PCB mounting brace and connecting the pogo pin electrical leads from the electrical connector on the base of the brace, through the PCB access opening, to a second side of the PCB;
   providing a light pipe sub-assembly including a light pipe housing having a button opening centrally formed therein, attachment arms extending outward thereof, and a plurality of light pipes, and snapping a push button into the button opening;
   attaching a spring to the power switch post and snapping the attachment arms of the light pipe sub-assembly through the PCB and onto to the PCB mounting brace, thus attaching the light pipe sub-assembly to both the PCB and the PCB mounting brace and compressing the spring; and
   snapping a status indicator lens onto the light pipe sub-assembly, thus defining a PCB assembly.
Statement 23. The method according to Statement 22, wherein the first side of the PCB includes a plurality of LEDs.
Statement 24. The method according to Statement 23, wherein the step of snapping the attachment arms of the light pipe sub-assembly through the PCB and onto to the PCB mounting brace further includes positioning the plurality of light pipes such that they extend between the plurality of LEDs and the status indicator lens and are configured to project light from the plurality of LEDs to the status indicator lens.
Statement 25. The method according to Statement 24, further including mounting the PCB assembly in a portion of an insect repeller housing.
Statement 26. The method according to Statement 24 or 25, wherein the insect repeller housing including a housing base assembly having a base and a main housing portion, and a two-part cap assembly having a cap base and a cap top, and wherein the PCB assembly is mounted in the housing base assembly.
Statement 27. The method according to Statement 26, further including mounting a heater module in the cap assembly, wherein the heater module includes:
   a heater PCB mounted between a heater base and a heater housing, wherein the heater base and the heater housing are attached via a snap-fit connection, and wherein the heater base includes three legs extending outwardly from a lower surface of the heater base;
   a cylindrical heater electrically connected to the heater PCB via electrical connectors;
   a cylindrical heater seal press mounted circumferentially around a first end of the heater; and
   a heater chimney attached to the heater seal opposite the heater, such that the heater chimney extends outwardly of a centrally formed opening in the cap top.

The principle and mode of operation of this invention have been explained and illustrated in its preferred embodiment. However, it must be understood that this invention may be practiced otherwise than as specifically explained and illustrated without departing from its spirit or scope.

## Claims

1. A printed circuit board (PCB) assembly for an insect repeller comprising:
a PCB mounting brace having a base and a PCB access opening formed therethrough;
a PCB having a first and a second side, the PCB having a microcontroller, one or more functional electronic components, and a power switch having an outwardly extending power switch post electrically mounted to the first side of the PCB and configured to operate the insect repeller, the PCB mounted within a groove formed in the PCB mounting brace;
a plurality of first pogo pins mounted to a first end of the PCB;
an electrical connector mounted to a base of the PCB mounting brace, the electrical connector having a plurality of second pogo pins and electrical leads extending from the electrical connector through the PCB access opening, to the second side of the PCB;
a light pipe sub-assembly including a light pipe housing having a button opening centrally formed therein, attachment arms extending outward thereof, and a plurality of light pipes, the attachment arms extending through the PCB and into the PCB mounting brace, a power button mounted within the button opening, a status indicator lens attached to the light pipe sub-assembly, and a spring mounted between the power switch post and the power button.

2. The PCB assembly according to claim 1, further including a plurality of light sources mounted to the first side of the PCB.

3. The PCB assembly according to claim 2, wherein the light sources are LEDs.

4. The PCB assembly according to claim 3, wherein the plurality of light pipes extends between the first side of the PCB and the status indicator lens, the plurality of light pipes configured to project light from the LEDs to the status indicator lens.

5. The PCB assembly according to claim 4, wherein the PCB is mounted in an insect repeller housing, the insect repeller housing including a housing base assembly having a base and a main housing portion, and a two-part cap assembly having a cap base and a cap top.

6. The PCB assembly according to claim 5, wherein the insect repeller housing further includes a heater assembly mounted in the cap assembly.

7. The PCB assembly according to claim 6, wherein the heater assembly includes
a heater PCB mounted between a heater base and a heater housing, wherein the heater base and the heater housing are attached via a snap-fit connection, and wherein the heater base includes three legs extending outwardly from a lower surface of the heater base;
a cylindrical heater electrically connected to the heater PCB via electrical connectors;
a cylindrical heater seal press mounted circumferentially around a first end of the heater; and
a heater chimney attached to the heater seal opposite the heater, such that the heater chimney extends outwardly of a centrally formed opening in the cap top.

8. The PCB assembly according to claim 7, wherein the base includes a battery housing configured to support at least one battery, and wherein the battery housing includes a cradle having a battery mounting surface, and an array of strengthening support ribs.

9. The PCB assembly according to claim 8, wherein the second pogo pins extend outwardly of an opening in the base and are configured for connection to a corresponding electrical connector in a docking station, the docking station configured to provide electrical power to an insect repeller within which the PCB assembly is mounted.

10. A method of assembling a printed circuit board (PCB) assembly for an insect repeller, the method comprising:
mounting to a first side of a PCB electrical components necessary to operate the insect repeller, including at least one of a microcontroller, one or more functional electronic components, a power button having an outwardly extending power switch post electrically mounted to the first side of the PCB and configured to operate the insect repeller;
placing a plurality of first pogo pins onto posts and heat staking the posts to lock the plurality of pogo pins in place at a first end of the PCB;
mounting an electrical connector to a base of a PCB mounting brace, the electrical connector having a plurality of second pogo pins and pogo pin electrical leads extending from the electrical connector;
mounting the PCB into a groove formed in the PCB mounting brace and connecting the pogo pin electrical leads from the electrical connector on the base of the brace, through the PCB access opening, to a second side of the PCB;
providing a light pipe sub-assembly including a light pipe housing having a button opening centrally formed therein, attachment arms extending outward thereof, and a plurality of light pipes, and snapping a push button into the button opening;
attaching a spring to the power switch post and snapping the attachment arms of the light pipe sub-assembly through the PCB and onto to the PCB mounting brace, thus attaching the light pipe sub-assembly to both the PCB and the PCB mounting brace and compressing the spring; and
snapping a status indicator lens onto the light pipe sub-assembly, thus defining a PCB assembly.

11. The method according to claim 10, wherein the first side of the PCB includes a plurality of LEDs.

12. The method according to claim 11, wherein the step of snapping the attachment arms of the light pipe sub-assembly through the PCB and onto to the PCB mounting brace further includes positioning the plurality of light pipes such that they extend between the plurality of LEDs and the status indicator lens and are configured to project light from the plurality of LEDs to the status indicator lens.

13. The method according to claim 12, further including mounting the PCB assembly in a portion of an insect repeller housing.

14. The method according to claim 12 or 13, wherein the insect repeller housing including a housing base assembly having a base and a main housing portion, and a two-part cap assembly having a cap base and a cap top, and wherein the PCB assembly is mounted in the housing base assembly.

15. The method according to claim 14, further including mounting a heater module in the cap assembly, wherein the heater module includes:
a heater PCB mounted between a heater base and a heater housing, wherein the heater base and the heater housing are attached via a snap-fit connection, and wherein the heater base includes three legs extending outwardly from a lower surface of the heater base;
a cylindrical heater electrically connected to the heater PCB via electrical connectors;
a cylindrical heater seal press mounted circumferentially around a first end of the heater; and
a heater chimney attached to the heater seal opposite the heater, such that the heater chimney extends outwardly of a centrally formed opening in the cap top.
